(19) Europäisches Patentamt
European Patent Office
Office européen des brevets

(11) **EP 3 848 115 A1**

(12) **EUROPEAN PATENT APPLICATION**
published in accordance with Art. 153(4) EPC

(43) Date of publication:
**14.07.2021 Bulletin 2021/28**

(51) Int Cl.:
***B01J 20/06*** (2006.01)      ***B01D 15/00*** (2006.01)

(21) Application number: **19857178.8**

(86) International application number:
**PCT/JP2019/033125**

(22) Date of filing: **23.08.2019**

(87) International publication number:
**WO 2020/050068 (12.03.2020 Gazette 2020/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **06.09.2018   JP 2018166842**
**07.11.2018   JP 2018209884**

(71) Applicants:
• **Nikkiso Co., Ltd.**
  **Shibuya-ku, Tokyo 150-6022 (JP)**
• **Tohoku University**
  **Sendai-shi, Miyagi 980-8577 (JP)**

(72) Inventors:
• **YOSHIOKA Toshiaki**
  **Sendai-shi Miyagi 980-8577 (JP)**
• **KAMEDA Tomohito**
  **Sendai Miyagi 980-8577 (JP)**
• **KITAGAWA Fumihiko**
  **Kanazawa-shi Ishikawa 920-0177 (JP)**
• **JIMBO Yoichi**
  **Kanazawa-shi Ishikawa 920-0177 (JP)**
• **KONDO Masayuki**
  **Kanazawa-shi Ishikawa 920-0177 (JP)**

(74) Representative: **Algemeen Octrooi- en Merkenbureau B.V.**
**P.O. Box 645**
**5600 AP Eindhoven (NL)**

(54) **LACTIC ACID ABSORBER AND METHOD FOR REMOVING LACTIC ACID**

(57)   A lactic acid absorber 4 includes at least one substance selected from the group consisting of layered double hydroxides, layered double oxides, weakly basic negative ion exchange resins having styrene-based dimethylamino groups, and strongly basic negative ion exchange resins.

FIG. 1A

EP 3 848 115 A1

FIG. 1B

FIG. 1C

FIG. 1D

EP 3 848 115 A1

**Description**

[TECHNICAL FIELD]

The present invention relates to a lactic acid adsorbent and a method for removing lactic acid.

[BACKGROUND ART]

In recent years, in fields such as pharmaceutical manufacturing and regenerative medicine, it has been required to artificially and efficiently mass-culture cells and microorganisms. Examples of cells for which mass culture is required include antibody-producing cells such as Chinese hamster ovary cells (CHO cells); and pluripotent stem cells such as embryonic stem cells (ES cells) and induced pluripotent stem cells (iPS cells). If these cells and the like can be stably cultured in large quantities for a long period of time, it becomes possible to efficiently produce biological substances such as monoclonal antibodies and differentiation-inducing tissues derived from pluripotent stem cells.

As a method for industrially mass-culturing cells and the like, suspension stirred culture with use of a culture tank such as a spinner flask may be feasible. The suspension stirred culture, however, tends to need large scale of equipment. It is, therefore, effective to increase the culture density of cells and the like in order to reduce costs. It is, however, known that increasing the culture density suppresses the proliferation of cells and the like. This is because the concentration of waste products (metabolites) in the culture solution (liquid medium) increases due to densification of the cells and the like, which reduces proliferative activity of the cells and the like. Lactic acid is known as a representative waste product that affects cells and the like.

It is therefore desirable to remove lactic acid accumulated in the culture solution, for stable growth of the cells in a high density condition. On the other hand, Patent Literature 1 for example discloses a cell culture apparatus in which a cell culture tank and a component adjusting solution tank are connected by a liquid feeding line provided with a culture solution component adjustment membrane that allows components to permeate depending on concentration difference. In this cell culture device, the waste products accumulated in the culture solution move to the component adjusting solution side, so that the concentration in the culture solution decreases. At the same time, the nutrients whose concentration has decreased during the culture are transferred from the component adjusting solution to the culture solution, and are replenished. The environment in the culture solution is thus maintained in a condition suitable for cell culture. The culture solution itself has been used as the component adjusting solution.

[Patent Literature]

[Patent Literature 1] WO2015/122528

[SUMMARY OF INVENTION]

[TECHNICAL PROBLEM]

The cell culture apparatus disclosed in Patent Literature 1 has removed waste products from the culture solution by use of the principle of dialysis. In order to attain sufficient removal of waste products, the capacity of the component adjusting solution tank has therefore been set to 10 times or more the capacity of the cell culture tank. There is therefore a problem that the required liquid amounts huge and becomes costly. In particular, in a case where the culture solution itself is used as the component adjusting solution, a large amount of expensive medium is consumed, which further increases the cost. In addition, in a case where the waste products are removed by using dialysis technology, there is also a problem that the structure of the culture apparatus becomes complicated.

In addition, lactic acid is often desired to be removed not only from the culture solution of cells and the like, but also from other solution systems. A novel lactic acid removal technique using a technique other than the dialysis technique has therefore been strongly desired.

The present invention has been made in view of these circumstances, and an object of which is to provide a novel lactic acid removing technique.

[SOLUTION TO PROBLEM]

Aimed at solving the aforementioned problem, one aspect of the present invention relates to a lactic acid adsorbent. This lactic acid adsorbent contains at least a substance selected from the group consisting of layered double hydroxide, layered double oxide, weakly basic anion exchange resins having styrene-bound dimethylamino group, and strongly basic anion exchange resin. According to this aspect, a novel lactic acid removal technique can be provided.

3

In the above aspect, the lactic acid adsorbent may come into contact with a solution containing lactic acid, to adsorb lactic acid in the solution. In addition, the solution may be a culture solution of cells or microorganisms that contains glucose. The strongly basic anion exchange resin may also contain at least one of strongly basic anion exchange resin having a styrene-bound trimethylammonium group or a strongly basic anion exchange resin having a styrene-bound dimethylethanolammonium group. Further, the weakly basic anion exchange resin may be of a high porous type or of MR type. The amount of addition of the lactic acid adsorbent to the solution may be more than 0.0005 g/mL and less than 0.2 g/mL.

Another aspect of the present invention relates to a method for removing lactic acid. The removing method includes bringing the lactic acid adsorbent of any of the above aspects into contact with lactic acid.

Note that also free combinations of these constituents, and also any of the constituents and expressions exchanged among the method, device and system, are valid as the aspects of the present invention.

[ADVANTAGEOUS EFFECTS OF INVENTION]

According to the present invention, a novel lactic acid removing technique can be provided.

[BRIEF DESCRIPTION OF DRAWINGS]

Fig. 1(A) to fig. 1(D) are schematic drawings for explaining methods for removing lactic acid according to embodiments.

Fig. 2 is a chart summarizing rates of lactic acid adsorption and rates of glucose adsorption of the lactic acid adsorbents in an aqueous solution containing lactic acid and glucose, when layered double hydroxide and layered double oxide are used as the lactic acid adsorbent.

Fig. 3 is a chart summarizing rates of lactic acid adsorption and rates of glucose adsorption of the lactic acid adsorbents in a cell culture solution, when layered double hydroxide and layered double oxide are used as the lactic acid adsorbent.

Fig. 4(A) is a chart summarizing rates of lactic acid adsorption and rates of glucose adsorption of the lactic acid adsorbents in an aqueous lactic acid solution and aqueous glucose solution, when layered double hydroxide is used as the lactic acid adsorbent. Fig. 4(B) is a chart summarizing rates of lactic acid adsorption and rates of glucose adsorption of the lactic acid adsorbents in the cell culture solution, when the layered double hydroxide is used as the lactic acid adsorbent.

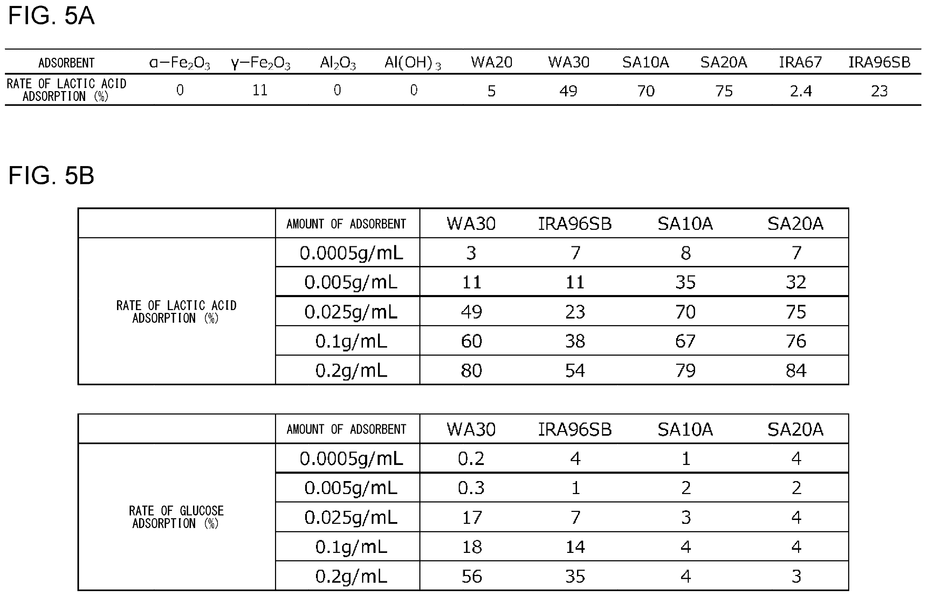

Fig. 5(A) is a chart summarizing rates of lactic acid adsorption of the lactic acid adsorbents in an aqueous solution containing lactic acid and glucose, when weakly basic anion exchange resin and strongly basic anion exchange resin are used as the lactic acid adsorbent. Fig. 5 (B) is a chart summarizing rates of lactic acid adsorption and rates of glucose adsorption of the lactic acid adsorbents in an aqueous solution containing lactic acid and glucose, when styrene-bound dimethylamine-type weakly basic anion exchange resin, and strongly basic anion exchange resin are used as the lactic acid adsorbent.

Fig. 6 is a chart summarizing rates of lactic acid adsorption and rates of glucose adsorption of lactic acid adsorbents in a cell culture solution, when styrene-bound dimethylamine type weakly basic anion exchange resin, and strongly basic anion exchange resin are used as the lactic acid adsorbent.

[DESCRIPTION OF EMBODIMENTS]

The present invention will be explained below on the basis of preferred embodiments, referring to the attached drawings. The embodiments are merely illustrative, and are not restrictive about the invention. All features and combinations thereof described in the embodiments are not always necessarily essential to the present invention. All constituents, members and processes illustrated in the individual drawings will be given same reference numerals, so as to properly avoid redundant explanations. Reduced scales and shapes of the individual parts in the individual drawings are properly given for simplicity of explanation, and should not be interpreted restrictively unless otherwise specifically noted. Also note that ordinal terms "first", "second" and so on used in this patent specification and in claims do not represent any sequential order or importance, and are used only for discrimination of one structure from the other. Further, in each drawing, some of the members that are not important for explaining the embodiment will be omitted.

The present inventors have made extensive studies on lactic acid removal technology, and have found an adsorbent capable of highly selectively adsorbing lactic acid. Specifically, the lactic acid adsorbent according to the present embodiment contains at least one substance selected from the group consisting of layered double hydroxide (LDH), layered double oxide (LDO), weakly basic anion exchange resin having styrene-bound dimethylamino group, and strongly basic anion exchange resin. The "styrene-bound dimethylamino group" means a structure in which a dimethylamino group, as an ion exchange group (functional group), is bound to a styrene skeleton. Hereinafter, a weakly basic anion exchange resin having a styrene-bound dimethylamino group is appropriately referred to as a styrene-bound dimethylamine-type weakly basic anion exchange resin. The same applies to anion exchange resins having other groups.

The layered double hydroxide is composed of octahedron-arrayed host layers positively charged due to $M^{3+}$ substituting a part of divalent metal $M^{2+}$ in $M(OH)_2$; and guest layers composed of anions that compensate the positive charge of the host layers, and intercalated water. Lactic acid (lactate ion) is adsorbed to LDH, as a result of ion exchange with the anions in the guest layer.

The layered double hydroxide is represented by the chemical formula below.

$$[M^{2+}_{1-x}M^{3+}_{x}(OH)_2][A^{n-}_{x/n} \cdot yH_2O]$$

In the formula, $M^{2+}$ represents a divalent metal ion selected from the group consisting of $C_u^{2+}$, $Mn^{2+}$, $Mg^{2+}$, $Fe^{2+}$, $Ca^{2+}$, $Ni^{2+}$, $Zn^{2+}$, $Co^{2+}$ and $Cd^{2+}$. $M^{3+}$ represents a trivalent metal ion selected from the group consisting of $Al^{3+}$, $Cr^{3+}$, $Fe^{3+}$, $Co^{3+}$, $In^{3+}$, $Mn^{3+}$ and $V^{3+}$. $A^{n-}$ represents an n-valent anion selected from the group consisting of $CO_3^{2-}$, $SO_4^{2-}$, $Cl^-$, $OH^-$, $SiO_4^{4-}$, $SO_4^{2-}$ and $NO_3^-$. x represents 0.22 to 0.3, n represents 1 to 3, and y represents 1 to 12.

The layered double oxide is oxide of LDH. LDO is obtainable by firing LDH at 200°C to 500°C, for example. Upon being brought in contact with aqueous lactic acid solution, LDO regenerates an LDH structure with lactic acid adsorbed between the layers.

Preferred examples of the layered double hydroxide and layered double oxide include Cu-Al-based LDH, Cu-Al-based LDO, Mg-Al-based LDH, Mg-Al-based LDO, and Ni-Al-based LDH. The notation "-based" means that the type of $A^{n-}$ in the above chemical formula is freely selectable. A plurality of types of layered double hydroxide and/or layered double oxide having different types of constituent metal ions and anions may be used in a mixed manner.

The weakly basic anion exchange resin having styrene-bound dimethylamino group is preferably of high porous type or of MR (macro-reticular) type. Examples of such a weakly basic anion exchange resin include high porous type WA30 (Mitsubishi Chemical Corporation) and MR type IRA96SB (Organo Corporation).

Examples of the strongly basic anion exchange resin include a strongly basic anion exchange resin having a styrene-bound trimethylammonium group and a strongly basic anion exchange resin having a styrene-bound dimethylethanolammonium group.

The styrene-bound trimethylammonium type strongly basic anion exchange resin is exemplified by gel type SA10A, SA12A, SA11A and NSA100 (all from Mitsubishi Chemical Corporation); and porous type PA306S, PA308, PA312, PA316, PA318L and HPA25 (all from Mitsubishi Chemical Corporation). Examples of the styrene-bound dimethylethanolammonium-type strongly basic anion exchange resin include SA20A and SA21A (all from Mitsubishi Chemical Corporation); and PA408, PA412 and PA418 (all from Mitsubishi Chemical Corporation).

The anion exchange resins are classified by shape into gel type, porous type, high porous type and MR type ones. The gel type one is composed of a polymer obtained by polymerizing a starting material of resin (monomer) in a solvent, and has a uniform three-dimensional structure. The gel type one has micropores only. The porous type and the high porous type ones are composed of a porous structure in which pores (macropores) are physically provided in the three-dimensional structure of the gel type one. The high porous type one is more porous than the porous type one. The MR type one is produced by improving a polymerization method for synthesizing the gel type one, and has specific surface area and pore volume larger than those of the gel type one. Since the weakly basic anion exchange resin has smaller adsorption capacity as compared with the strongly basic anion exchange resin, so that preferred are the high porous type or MR type one having a large adsorption area. On the other hand, the strongly basic anion exchange resin has larger adsorptive capacity as compared with the weakly basic anion exchange resin, so that any of gel type, porous type, high porous type and MR type structures is acceptable.

The weakly basic anion exchange resin and the strongly basic anion exchange resin can adsorb lactic acid, as a result of electric interaction between a free functional groups and lactate ion, or ion exchange between an exchangeable ion that interacts with the functional group, and lactate ion. Aforementioned SA10A, SA12A, SA11A, NSA100, PA306S, PA308, PA312, PA316, PA318L, HPA25, SA20A, SA21A, PA408, PA412 and PA418 adsorb lactic acid, by ion exchange between exchangeable ion $Cl^-$ and lactate ion. WA30 and IRA96SB adsorb lactic acid, on the basis of electrical interaction between free functional groups and lactate ion. The lactic acid adsorbent may contain any freely selectable combination of the layered double hydroxides, the layered double oxides, the styrene-bound dimethylamine-type weakly basic anion exchange resin and the strongly basic anion exchange resins.

By bringing the lactic acid adsorbent, containing at least one substance selected from the group consisting of layered double hydroxides, layered double oxides, weakly basic anion exchange resins having styrene-bound dimethylamino group, and strongly basic anion exchange resin, into contact with lactic acid, lactic acid can now be adsorbed to the substance. In particular, the lactic acid adsorbent of the present embodiment can be suitably used when adsorbing and removing lactic acid in solution. In this case, lactic acid in the solution can be adsorbed by bringing the lactic acid adsorbent into contact with the lactic acid-containing solution. Lactic acid in the solution can thus be removed.

In addition in a case where the solution is a culture solution of cells or microorganisms that contains glucose, the lactic acid adsorbent can adsorb lactic acid to be removed, more selectively than glucose to be remained in the culture solution is adsorbed. Further, the lactic acid adsorbent is preferably chosen from those less toxic to cells and microorganisms. The type of culture solution is not particularly limited.

The amount of addition of the lactic acid adsorbent to the solution, in other words, the concentration of the lactic acid adsorbent in the solution is preferably more than 0.0005 g/mL, and more preferably 0.005 g/mL or more. The amount of addition is preferably less than 0.2 g/mL, and more preferably 0.1 g/mL or less. With the amount of addition of the lactic acid adsorbent adjusted to more than 0.0005 mg/mL, the rate of lactic acid adsorption can be increased more exactly. Further, with the amount of addition adjusted to 0.005 g/mL or more, a rate of lactic acid adsorption of 40% or more is attainable in a water-based solution. Even in the culture solution, a rate of lactic acid adsorption of 10% or more is attainable. The amount of lactic acid in the solution can thus be reduced more exactly. The rate of lactic acid adsorption is the ratio of the amount of adsorbed lactic acid, to the total amount of lactic acid in the solution.

With the amount of addition of the lactic acid adsorbent adjusted to less than 0.2 g/mL, the rate of glucose adsorption can be suppressed more exactly. With the amount of addition further adjusted to 0.1g/mL or less, a rate of glucose adsorption of 20% or less is attainable in the culture solution. Meanwhile in a water-based solution, the rate of glucose adsorption can be suppressed to about 1/3 of the rate of lactic acid adsorption. This can more exactly suppress glucose from being reduced possibly caused by the lactic acid adsorbent. Therefore, cells and the like can be cultured more efficiently. The rate of glucose adsorption is the ratio of the amount of adsorbed glucose to the total amount of glucose in the solution.

The cells and microorganisms cultured using the culture solution are not particularly limited. For example, cultured cells include pluripotent stem cells such as human iPS cells, human ES cells, and human Muse cells; somatic stem cells such as mesenchymal stem cells (MSC cells) and nephron progenitor cells; tissue cells such as human renal proximal tubule epithelial cells, human distal tubule epithelial cells, and human collecting duct epithelial cells; antibody-producing cell lines such as human fetal renal cells (HEK293 cells); antibody-producing cell lines derived from animals other than humans such as Chinese hamster ovary cells (CHO cells) and insect cells (SF9 cells). Since these cells are cells for which mass culture is particularly desired, they are more preferred targets to which the lactic acid adsorbent according to the present embodiment is applied.

(Method for Removing Lactic Acid)

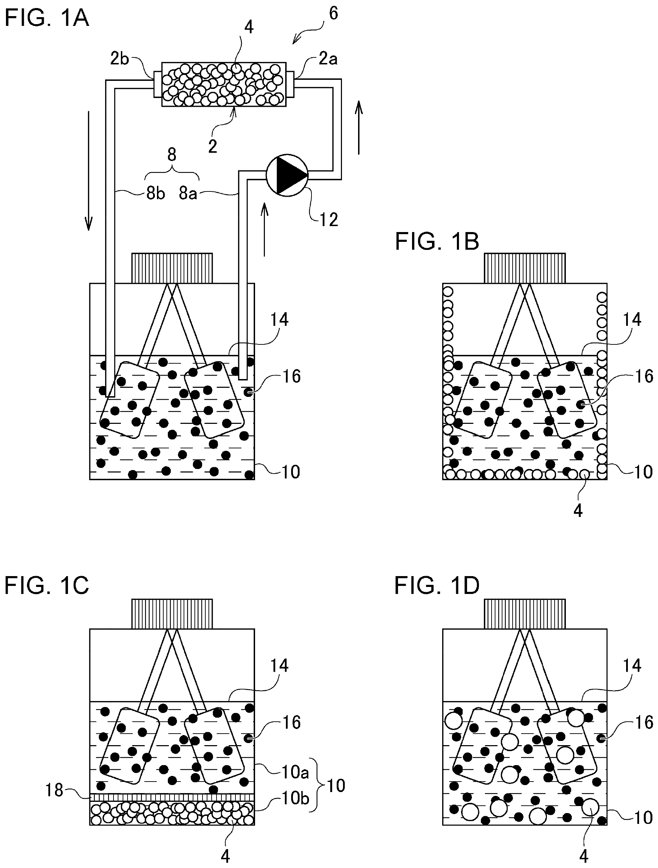

The method for removing lactic acid according to the present embodiment includes bringing the above-mentioned lactic acid adsorbent into contact with lactic acid (lactate ion). Preferably, the method involves bringing the lactic acid adsorbent into contact with a solution containing lactic acid. The method of bringing the lactic acid adsorbent into contact with lactic acid is exemplified as follows, although not particularly limited thereto. Fig. 1(A) to fig. 1(D) are schematic drawings for explaining methods for removing lactic acid according to the embodiment. In the following, lactic acid removal from the culture solution will be described as an example. Also removal of lactic acid from other solutions can be carried out in the same way.

In a first aspect as illustrated in Fig. 1(A), an adsorption module 6 having a container 2 such as a column packed with a lactic acid adsorbent 4 is prepared. The container 2 has an inlet 2a and an outlet 2b through which the inside and the outside of the container 2 are communicated. The lactic acid adsorbent 4 has, for example, a particle form. The adsorption module 6 is connected, through a circulation path 8, to a culture vessel 10 such as a spinner flask. The circulation path 8 includes an outward path 8a connecting the culture vessel 10 and the inlet 2a of the container 2, and a return path 8b connecting the outlet 2b of the container 2 and the culture vessel 10. A pump 12 is connected in the middle of the outward path 8a. The culture solution 14 and the cells 16 are housed in the culture vessel 10. The pump 12 may alternatively be arranged on the return path 8b.

When the pump 12 operates, the culture solution 14 is sucked from the culture vessel 10 and sent into the container 2 of the adsorption module 6 via the outward path 8a. The culture solution 14 fed into the container 2 is returned to the culture vessel 10 through the return path 8b. The culture solution 14 comes into contact with the lactic acid adsorbent 4 packed in the container 2, in the process of circulating between the culture vessel 10 and the adsorption module 6. In this process, lactic acid in the culture solution 14 is adsorbed by the lactic acid adsorbent 4. Lactic acid in the culture solution 14 is thus removed. A filter (not illustrated) is provided at the end of the outward path 8a on the side connected to the culture vessel 10. The cells 16 are consequently suppressed from flowing towards the adsorption module 6. In the process of circulating the culture solution 14 between the culture vessel 10 and the adsorption module 6, medium components such as glucose and protein necessary for culturing the cells 16 may be replenished into the culture solution 14.

That is, in the first aspect, lactic acid in the culture solution 14 is removed by using the culture apparatus equipped with the adsorption module 6 having the lactic acid adsorbent 4, the culture vessel 10 containing the cells or microorganisms and the culture solution 14, and the circulation path 8 that connects the adsorption module 6 and the culture vessel 10, so as to allow the culture solution 14 to circulate therethrough.

In a second aspect illustrated in Fig. 1(B), the lactic acid adsorbent 4 is supported on the inner wall surface of the culture vessel 10. The culture vessel 10 houses the culture solution 14 and the cells 16. The culture solution 14 therefore comes into contact with the lactic acid adsorbent 4 exposed on the inner wall surface of the culture vessel 10. The lactic acid in the culture solution 14 can thus be adsorbed to the lactic acid adsorbent 4. The culture vessel 10 is exemplified by spinner flask, petri dish, well plate, cell culture insert, and microsphere.

Method for supporting the lactic acid adsorbent 4 on the inner wall surface of the culture vessel 10 is exemplified by a method of adhering the lactic acid adsorbent 4 to the inner wall surface of the culture vessel 10, or, a method of molding the culture vessel 10, if it were made of resin, by using a resin preliminarily mixed with the lactic acid adsorbent 4. That is, in the second aspect, lactic acid in the culture solution 14 is removed by using the culture apparatus that includes the culture vessel 10 and the lactic acid adsorbent 4 supported on the inner wall surface of the culture vessel 10.

In a third aspect as illustrated in Fig. 1(C), the culture vessel 10 has a structure in which the inside of the vessel is divided into an upper part 10a and a lower part 10b by a diaphragm 18 such as a porous membrane. Such a culture vessel 10 is exemplified by a cell culture insert. The upper part 10a houses the culture solution 14 and the cells 16, and the lower part 10b houses the culture solution 14 and the lactic acid adsorbent 4. The culture solution 14 can move back and forth between the upper part 10a and the lower part 10b through the diaphragm 18. In contrast, the cells 16 and the lactic acid adsorbent 4 cannot pass through the diaphragm 18.

In such a structure, the culture solution 14 comes into contact with the lactic acid adsorbent 4 housed in the lower part 10b. The lactic acid in the culture solution 14 can thus be adsorbed to the lactic acid adsorbent 4. That is, in the third aspect, lactic acid in the culture solution 14 is removed by using the culture apparatus provided with the culture vessel 10, the lactic acid adsorbent 4, and the diaphragm 18 that divides the culture vessel 10 into a first space housing the lactic acid adsorbent 4, and a second space housing the cells 16.

In a fourth aspect illustrated in Fig. 1(D), the particulate lactic acid adsorbent 4 is allowed to disperse, precipitate or suspend in the culture solution 14. The lactic acid in the culture solution 14 can thus be adsorbed to the lactic acid adsorbent 4. The lactic acid adsorbent 4 preferably has a predetermined size or larger, for example 10 $\mu$m or larger, in view of preventing phagocytosis by the cells 16. That is, in the fourth aspect, lactic acid in the culture solution 14 is removed by using a culture apparatus provided with the culture vessel 10, and the lactic acid adsorbent 4 added to the culture solution 14 in the culture vessel 10.

The lactic acid adsorbent is preferably coated with a resin such as polyvinyl alcohol or alginic acid; and a biological gel such as collagen or gelatin, or the like.

This suppresses outflow of fine particles that may affect cells and the like, out from the lactic acid adsorbent into the culture solution. The lactic acid adsorbent is alternatively formed by kneading ceramic binder, resin binder, biological gel or the like, with the layered double hydroxide and/or layered double oxide. This also makes it possible to suppress the outflow of fine particles. Examples of the ceramic binder include alumina binder and colloidal silica. Examples of the resin binder include alginic acid, polyvinyl alcohol, and carboxymethyl cellulose. Examples of the biological gel include collagen and gelatin.

When detecting the lactic acid concentration in the solution, it is preferable to use a medium component analyzer, although the method for detection is not particularly limited. The lactic acid concentration can alternatively be detected by colorimetry by use of a predetermined measuring reagent, enzyme electrode method utilizing the substrate specificity of enzyme, high performance liquid chromatography (HPLC), or the like.

As described above, the lactic acid adsorbent according to the present embodiment contains at least one substance selected from the group consisting of layered double hydroxide, layered double oxide, weakly basic anion exchange resin having styrene-bound dimethylamino group, and strongly basic anion exchange resin. In addition, the method for removing lactic acid according to the present embodiment includes bringing this lactic acid adsorbent into contact with lactic acid. This makes it possible to remove lactic acid without using a huge amount of solution, unlike a known case where lactic acid is removed by dialysis technique. The present embodiment can therefore provide a novel lactic acid removing technique capable of removing lactic acid at low cost. Further, lactic acid can be removed only by bringing the lactic acid adsorbent into contact with the lactic acid-containing solution, thus enabling the present embodiment to simplify structure of the culture apparatus.

Further, in a case where the solution is culture solution of cells, the amount of consumption of the culture solution can be reduced as compared with known dialysis techniques. Since the culture solution is generally expensive, so that the cost can be further reduced. In addition, cells and the like can be mass-cultured at high density by removing lactic acid. It also becomes possible to suppress pH of the medium from decreasing due to lactic acid, enabling mass culturing of cells at high density. Furthermore, in a case where the cells are pluripotent stem cells, the removal of lactic acid not only enables high-density mass culture of the cells, but can also keep the cells in an undifferentiated state, that is, the cells can remain pluripotent (can keep pluripotency). This therefore enables to obtain a large amount of cells suitable for producing biological substances and producing differentiation-inducing tissues. The cost required for drug manufacturing and regenerative medicine can therefore be reduced.

In addition, the lactic acid adsorbent of the present embodiment can adsorb lactic acid, more selectively than glucose which is a useful component is adsorbed. This therefore enables more efficient cell culture. Hence, the lactic acid adsorbent of the present embodiment is particularly useful for removing lactic acid in the glucose-containing culture solution. The lactic acid adsorbent of the present embodiment may be used in combination with some adsorbent for other cell waste products.

The embodiments of the present invention have been described in detail above. The above-described embodiments merely illustrate specific examples in carrying out the present invention. Contents of the embodiments do not limit the technical scope of the present invention, instead allowing numerous design changes such as modification, addition, and deletion of constituents, without departing from the spirit of the present invention specified in the claims. Any new embodiment with design change will have effects derived both from an embodiment and modification to be combined. In the above-described embodiments, all contents possibly subject to such design change have been emphasized with a notation stating "... of the present embodiment" or "in the present embodiment". Also any content without such notation is, however, acceptable for the design change. Free combination of the above constituents is also valid as an aspect of the present invention.

[EXAMPLES]

Examples of the present invention will be explained below. Examples are merely illustrative, and by no means limit the present invention.

Layered Double Hydroxide and Layered Double Oxide

Synthesis of Lactic Acid Adsorbent

Synthesis of Layered Double Hydroxide Cu-Al LDH

First, a five-neck round-bottom flask containing deionized water was prepared, followed by stirring at conditions of 30°C and 300 rpm under nitrogen gas flow. To the deionized water, a 1.0 mol/L NaOH solution (Kanto Chemical Co., Inc.) was added dropwise to raise the pH to 8.0. Next, 250 mL of Cu-Al mixed solution was added dropwise to the deionized water at a rate of 10 mL/min, while stirring the content of the five-neck round-bottom flask at conditions of 30°C and 300 rpm. Also a 1.0 mol/L NaOH solution was concurrently added dropwise into the flask, to maintain the pH of the aqueous solution at 8.0. After completion of the dropwise addition of the Cu-Al mixed solution, the suspension was stirred for one hour, and then filtered under suction. The product was then washed with deionized water, until the filtrate became neutral. The obtained product was dried under reduced pressure at 40°C for 40 hours, and then crushed to obtain Cu-Al LDH.

Synthesis of Layered Double Oxide Cu-Al LDO

The Cu-Al LDH prepared according to the aforementioned procedures was calcined in an electric furnace at 200°C for 4 hours, to obtain Cu-Al LDO.

Synthesis of Layered Double Oxide Mg-Al LDO

First, a five-neck round-bottom flask containing 500 mL of an aqueous $Na_2CO_3$ solution was prepared. The content was then stirred at conditions of 30°C and 300 rpm. During the stirring, 500 mL of Mg-Al mixed solution was added dropwise to the aqueous solution in the flask at a rate of 15 mL/min. Also an aqueous 1.25 mol/L NaOH solution was concurrently added dropwise into the flask, to maintain the pH of the aqueous solution at $10.5 \pm 0.1$. After completion of the dropwise addition of the Mg-Al mixed solution, the suspension was stirred for one hour, and then filtered under suction. The obtained product was dried under reduced pressure at 40°C for 40 hours, crushed, and then calcined at 500°C for 2 hours in an electric furnace, to obtain Mg-Al LDO.

Synthesis of Layered Double Hydroxide Mg-Al LDH

First, a five-neck round-bottom flask containing deionized water was prepared. The content was then stirred at conditions of 30°C and 300 rpm under nitrogen gas flow. During the stirring, 500 mL of Mg-Al mixed solution was added dropwise to the deionized water in the flask at a rate of 15 mL/min. Also an aqueous 1.25 mol/L NaOH solution was concurrently added dropwise into the flask, to maintain the pH of the aqueous solution at $10.5 \pm 0.1$. After completion of the dropwise addition of the Mg-Al mixed solution, the suspension was stirred for one hour, and then filtered under suction. The obtained product was dried under reduced pressure at 40°C for 40 hours, and then crushed to obtain Mg-Al LDH.

Synthesis of Layered Double Hydroxide Ni-Al LDH

First, a five-neck round-bottom flask containing deionized water was prepared. The content was then stirred at conditions of 30°C and 300 rpm under nitrogen gas flow. To the deionized water, a 1.0 mol/L NaOH solution (Kanto Chemical Co., Inc.) was added dropwise to deionized water to raise the pH to 10.5. Next, 250 mL of Ni-Al mixed solution was added dropwise to the deionized water at a rate of 10 mL/min, while stirring the content of the five-neck round-bottom flask at conditions of 30°C and 300 rpm. Also a 1.0 mol/L NaOH solution was concurrently added dropwise into the flask, to maintain the pH of the aqueous solution at 10.5. After completion of the dropwise addition of the Ni-Al mixed solution, the suspension was stirred for one hour, and then filtered under suction. The product was then washed with deionized water, until the filtrate became neutral. The obtained product was dried under reduced pressure at 40°C for 40 hours, and then crushed to obtain Ni-Al LDH.

Performance Analyses of Adsorbents in Aqueous Solution

Containing Lactic Acid and Glucose (Water-Based Solution) 1

Lactic acid (Kanto Chemical Co., Inc.) and glucose (Kanto Chemical Co., Inc.) were added to pure water, to prepare an aqueous solution with a glucose concentration of 1000 ppm and a lactic acid concentration of 10 mM. Aqueous sodium hydroxide solution was further added to the aqueous solution, to adjust the pH of the aqueous solution to 7.2. Then, 20 mL each of the aqueous solution was dispensed into a plurality of 50 mL Erlenmeyer flasks. Also 0.5 g each of various adsorbents was added to the aqueous solution in each flask. The concentration of the adsorbent was therefore 0.025 g/mL. The mixture was then shaken at 37°C and 150 rpm for 24 hours.
The adsorbent employed here includes spherical activated carbon (SAC: Kureha Corporation), metal oxide (MgO: Kanto Chemical Co., Ltd.), ceramic ($SiO_2$: Kanto Chemical Co., Ltd.), Cu-Al LDH, Cu-Al LDO and Mg-Al LDO.
After 24 hours, the aqueous solution and the adsorbent were separated by a 0.1 $\mu$m filter. Lactic acid concentration and glucose concentration in the aqueous solution were then measured using an HPLC apparatus (JASCO Corporation). In addition, the rate of lactic acid adsorption and the rate of glucose adsorption of each adsorbent were calculated from the equation below.

```
Adsorption rate (%) = {[Concentration before adsorption

 - Concentration after adsorption]/Concentration before

adsorption} × 100
```

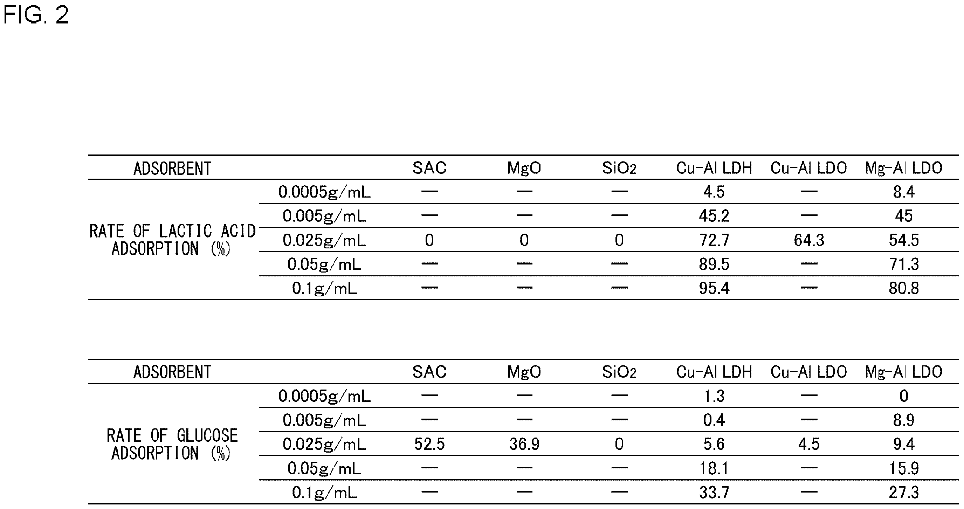

Results are summarized in Fig. 2. Fig. 2 is a chart summarizing the rate of lactic acid adsorption and the rate of glucose adsorption of the lactic acid adsorbents in an aqueous solution containing lactic acid and glucose, when the layered double hydroxide and the layered double oxide were used as the lactic acid adsorbent. As summarized in Fig. 2, spherical activated carbon (SAC), metal oxide (MgO) and ceramic ($SiO_2$) did not adsorb lactic acid at all at an adsorbent concentration of 0.025g/mL, but in contrast, each of Cu-Al LDH which is a layered double hydroxide, and Cu-Al LDO and Mg-Al LDO which are layered double oxides, was found to demonstrate a rate of lactic acid adsorption of 50% or higher. From this, the layered double hydroxide and the layered double oxides were confirmed to have excellent lactic acid adsorption ability.
In addition, while spherical activated carbon (SAC) and metal oxide (MgO) were found to demonstrate a rate of glucose adsorption of 35% or higher, the layered double hydroxide and the layered double oxides were found to demonstrate a rate of glucose adsorption of 10% or lower. From this, the layered double hydroxide and the layered double oxides were confirmed to adsorb lactic acid, more selectively than glucose is adsorbed.
Of the layered double hydroxide and the layered double oxides thus confirmed to be suitable as the lactic acid adsorbents, Cu-Al LDH and Mg-Al LDO were then subjected to the aforementioned adsorption test while varying the amount of addition to the aqueous solution, to calculate the rate of lactic acid adsorption and the rate of glucose adsorption. The amount of addition (concentration) was varied among 0.01 g (0.0005 g/mL), 0.1 g (0.005 g/mL), 0.5 g (0.025 g/mL), 1.0 g (0.05 g/mL) and 2.0 g (0.1 g/mL). Results are summarized in Fig. 2.
As summarized in Fig. 2, the adsorbents were confirmed to adsorb lactic acid at any adsorbent concentration.

The adsorbents were also confirmed to attain still higher rate of lactic acid adsorption, at a concentration exceeding 0.0005 g/mL, and further 0.005 g/mL or higher. It was also confirmed that the rate of lactic acid adsorption increased as the concentration of the adsorbent increased, but concurrently the rate of glucose adsorption also tended to increase. Cu-Al LDH, however, demonstrated the rate of lactic acid adsorption nearly three times as large as the rate of glucose adsorption, even at an adsorbent concentration of 0.1 g/mL where the rate of glucose adsorption peaked at 33.7%. Similarly, Mg-Al LDO demonstrated the rate of lactic acid adsorption nearly three times as large as the rate of glucose adsorption, even at an adsorbent concentration of 0.1 g/mL where the rate of glucose adsorption peaked at 27.3%. From this, the layered double hydroxide and the layered double oxides were confirmed to adsorb lactic acid, more selectively than glucose is adsorbed. Performance Analyses of Adsorbents in Cell Culture Solution 1

Sodium lactate (FUJIFILM Wako Pure Chemical Corporation) was added to a pluripotent stem cell medium (StemFit AK02N: Ajinomoto Co., Inc.) to prepare a medium having a glucose concentration of 250 mg/mL and a lactic acid concentration of 10 mM. Twenty milliliters each of the medium was dispensed into each of a plurality of 50 mL tubes (Thermo Fisher Scientific Inc.). Each of various adsorbents was then added to the medium in each tube. Adsorbents used include Cu-Al LDH, Cu-Al LDO and Mg-Al LDO. The amount of addition (concentration) of the adsorbent was varied among 0.01 g (0.0005 g/mL), 0.1 g (0.005 g/mL), 0.5 g (0.025 g/mL), 1.0 g (0.05 g/mL), 2.0 g (0.1 g/mL) and 4.0 g (0.2 g/mL). The mixture was then shaken at 37°C, 60 rpm for 24 hours.

After 24 hours, the culture solution and the adsorbent were separated by a 0.22 $\mu$m filter. The lactic acid concentration and the glucose concentration in the cell culture solution were then measured by using a blood gas analyzer (ABL800 FLEX: Radiometer Medial ApS). The rate of lactic acid adsorption and the rate of glucose adsorption of each adsorbent were calculated from the equation above.

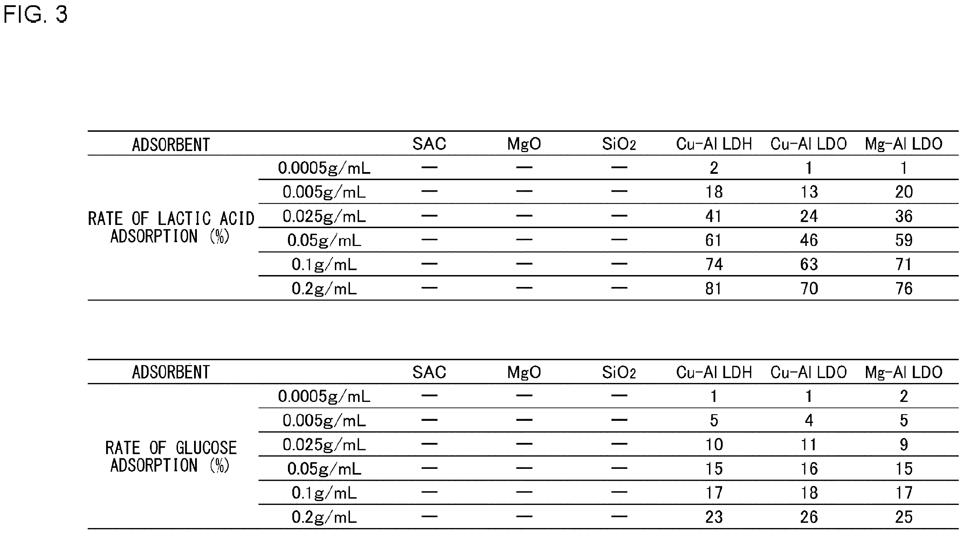

Results are summarized in Fig. 3. Fig. 3 is a chart summarizing the rate of lactic acid adsorption and the rate of glucose adsorption of the lactic acid adsorbents in the cell culture solution, when the layered double hydroxide and the layered double oxides were used as the lactic acid adsorbent. As summarized in Fig. 3, the layered double hydroxide and the layered double oxides were confirmed to adsorb lactic acid also in the cell culture solution, although becoming slightly lower than in the water-based solution. Further improved rate of lactic acid adsorption was confirmed to be attainable, particularly at an adsorbent concentration exceeding 0.0005 g/mL, and further at 0.005 g/mL or higher. Each of the layered double hydroxide and the layered double oxides was also found to demonstrate a rate of glucose adsorption of 26% at the maximum. From this, the layered double hydroxide and the layered double oxides were confirmed to be suitable for removing lactic acid in the medium that contains glucose.

It was confirmed that, also in the cell culture solution, the rate of lactic acid adsorption increased as the adsorbent concentration increased, but concurrently the rate of glucose adsorption also increased. It was however confirmed that the rate of glucose adsorption can be reduced more satisfactorily, if the adsorbent concentration is adjusted to lower than 0.2 g/mL, and preferably to 0.1 g/mL or lower.

Performance Analyses of Adsorbents in Aqueous Solution

Containing Lactic Acid and Glucose (Water-Based Solution) 2

The layered double oxides Mg-Al LDH and Ni-Al LDH, having not been used in the aforementioned Analyses 1, were subjected to performance analyses of adsorbents. First, sodium lactate (Fujifilm Wako Pure Chemical Corporation) and glucose (Kanto Chemical Co., Inc.) were individually added to pure water to prepare an aqueous lactic acid solution with a lactic acid concentration of 10 mM, and an aqueous glucose solution with a glucose concentration of 1000 ppm. Then, 20 mL each of the aqueous solutions were dispensed into separate 50 mL Erlenmeyer flasks. Also 0.5 g each of various adsorbents was added to the aqueous solution in each flask. The concentration of the adsorbent was therefore 0.025 g/mL. The mixture was then shaken at 37°C and 150 rpm for 24 hours. The adsorbed employed were Mg-Al LDH and Ni-Al LDH.

After 24 hours, the aqueous solution and the adsorbent were separated by a 0.1 $\mu$m filter. Lactic acid concentration and glucose concentration in the aqueous solution were then measured using an HPLC apparatus (JASCO Corporation). The rate of lactic acid adsorption and the rate of glucose adsorption of each adsorbent were calculated from the equation above.

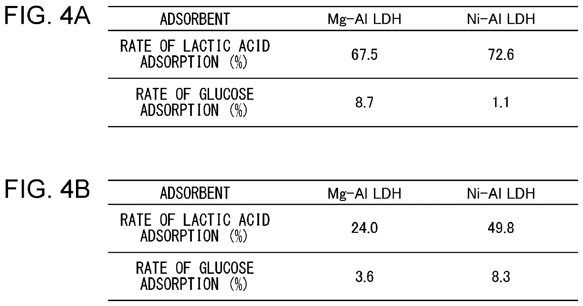

Results are summarized in Fig. 4(A). Fig. 4(A) is a chart summarizing the rate of lactic acid adsorption and the rate of glucose adsorption of the lactic acid adsorbents in the aqueous lactic acid solutions and aqueous glucose solution, when the layered double hydroxides were used as the lactic acid adsorbent. As summarized in Fig. 4, each of Mg-Al LDH and Ni-Al LDH was found to demonstrate a rate of lactic acid adsorption of as very high as 67.5% or above, at an adsorbent concentration of 0.025 g/mL. Meanwhile, each of Mg-Al LDH and Ni-Al LDH was found to demonstrate a rate of glucose adsorption of as very low as 8.7% or below. From this, the layered double hydroxides were confirmed to have excellent lactic acid adsorption ability, and can adsorb lactic acid more selectively than glucose is adsorbed. Performance Analyses of Adsorbents in Cell Culture Solution 2

Sodium lactate (FUJIFILM Wako Pure Chemical Corporation) was added to a pluripotent stem cell medium (StemFit AK02N: Ajinomoto Co., Inc.) to prepare a medium having a glucose concentration of 250 mg/mL and a lactic acid concentration of 10 mM. Ten milliliters each of the medium was then dispensed into a plurality of 15 mL tubes (Thermo Fisher Scientific). Each of various adsorbents was then added to the medium in each tube. The adsorbed employed were Mg-Al LDH and Ni-Al LDH. The amount of addition (concentration) of the adsorbents was adjusted to 0.25 g (0.025 g/mL). The mixture was then shaken at 37°C and 60 rpm for 24 hours.

After 24 hours, the culture solution and the adsorbent were separated by a 0.22 $\mu$m filter. The lactic acid concentration and the glucose concentration in the cell culture solution were then measured by using a blood gas analyzer (ABL800 FLEX: Radiometer Medial ApS). The rate of lactic acid adsorption and the rate of glucose adsorption of each adsorbent were calculated from the equation above.

Results are summarized in Fig. 4(B). Fig. 4(B) is a chart summarizing rates of lactic acid adsorption and rates of glucose adsorption of the lactic acid adsorbents in the cell culture solution, when the layered double hydroxide is used as the lactic acid adsorbent. As summarized in Fig. 4(B), Mg-Al LDH and Ni-Al LDH were confirmed to adsorb lactic acid also in the cell culture solution, although becoming slightly lower than in the water-based solution. Meanwhile, each of Mg-Al LDH and Ni-Al LDH demonstrated a rate of glucose adsorption of 8.3% at the maximum. From this, the layered double hydroxides were confirmed to be suitable for removing lactic acid in the medium that contains glucose.

Weakly Basic Anion Exchange Resin and Strongly Basic Anion

Exchange Resin

Performance Analyses of Adsorbents in Aqueous Solution

Containing Lactic Acid and Glucose (Water-Based Solution) 1

Lithium lactate (FUJIFILM Wako Pure Chemical Corporation) and glucose (Kanto Chemical Co., Inc.) were added to pure water, to prepare an aqueous solution having a lactic acid concentration of 10 mM and a glucose concentration of 1000 ppm. Aqueous sodium hydroxide solution was added to the aqueous solution, to adjust the pH of the aqueous solution to 7.2. Then, 20 mL each of the aqueous solution was dispensed into a plurality of 50 mL Erlenmeyer flasks. Also 0.5 g each of various adsorbents was added to the aqueous solution in each flask. The concentration of the adsorbent was therefore 0.025 g/mL. The mixture was then shaken at 37°C and 150 rpm for 24 hours.

The adsorbents used are as follows.

Metal Oxides and Metal Hydroxides:

$\alpha$-Fe$_2$O$_3$ (Soekawa Rikagaku Co., Ltd.)
$\gamma$-Fe$_2$O$_3$ (Soekawa Rikagaku Co., Ltd.)
Al$_2$O$_3$ (Kanto Chemical Co., Inc.)
Al(OH)$_3$ (Kanto Chemical Co., Ltd.)

Weakly Basic Anion Exchange Resins:

Styrene-bound polyamine type WA20 (Mitsubishi Chemical Corporation)
Styrene-bound dimethylamine type WA30 (Mitsubishi Chemical Corporation)
Acrylic dimethylamine type IRA67 (Organo Corporation)
Styrene-bound dimethylamine type IRA96SB (Organo Corporation)

Strongly Basic Anion Exchange Resins:

Styrene-bound trimethylammonium type SA10A (Mitsubishi Chemical Corporation)
Styrene-bound dimethylethanolammonium type SA20A (Mitsubishi Chemical Corporation)

Note that WA20 belongs to high porous type, WA30 belongs to high porous type, IRA67 belongs to gel type, IRA96SB belongs to MR type, SA10A belongs to gel type, and SA20A belongs to gel type.

After 24 hours, the aqueous solution and the adsorbent were separated by a 0.1 $\mu$m filter. Lactic acid concentration and glucose concentration in the aqueous solution were then measured using an HPLC apparatus (JASCO Corporation). In addition, the rate of lactic acid adsorption of each adsorbent was calculated from the equation above.

Results are summarized in Fig. 5(A). Fig. 5(A) is a chart summarizing the rate of lactic acid adsorption of the lactic acid adsorbent in an aqueous solution containing lactic acid and glucose, when the weakly basic anion exchange resins and the strongly basic anion exchange resins were used as the lactic acid adsorbent. As summarized in Fig. 5(A), metal oxides and metal hydroxides ($\alpha$-Fe$_2$O$_3$, $\gamma$-Fe$_2$O$_3$, Al$_2$O$_3$, Al(OH)$_3$), styrene polyamine-type weakly basic anion exchange resin (WA20) and acrylic dimethylamine-type weakly basic anion exchange resin (IRA67) were found to hardly adsorb lactic acid, at an adsorbent concentration of 0.025 g/mL.

In contrast, styrene-bound dimethylamine-type weakly basic anion exchange resins (WA30 and IRA96SB), styrene-bound trimethylammonium-type strongly basic anion exchange resin (SA10A), and styrene-bound dimethylethanolammonium-type strongly basic anion exchange resin (SA20A) were found to demonstrate the rate of lactic acid adsorption as good as 20% or higher. In particular, each of WA30, SA10A and SA20A attained a more excellent rate of lactic acid adsorption of 49% or above. From these results, the styrene-bound dimethylamine type weakly basic anion exchange resin and the strongly basic anion exchange resins were confirmed to have excellent lactic acid adsorption ability.

Performance Analyses of Adsorbents in Aqueous Solution Containing Lactic Acid and Glucose (Water-Based Solution) 2

WA30, IRA96SB, SA10A and SA20A, having demonstrated large capacities of lactic acid adsorption in Analyses 1 above, were subjected to the aforementioned adsorption test while varying the amount of addition (concentration) to the aqueous solution, and the rate of lactic acid adsorption and the rate of glucose adsorption were calculated. The concentrations were varied among 0.01 g (0.0005 g/mL), 0.1 g (0.005 g/mL), 0.5 g (0.025 g/mL), and 2.0 g (0.1 g/mL).

Results are summarized in Fig. 5(B). Fig. 5 (B) is a chart summarizing rates of lactic acid adsorption and rates of glucose adsorption of the lactic acid adsorbents in an aqueous solution containing lactic acid and glucose, when styrene-bound dimethylamine-type weakly basic anion exchange resin, and strongly basic anion exchange resin are used as the lactic acid adsorbent. As summarized in Fig. 5 (B), the adsorbents were confirmed to adsorb lactic acid at any adsorbent concentration. The adsorbents were also confirmed to attain still higher rate of lactic acid adsorption, at a concentration exceeding 0.0005 g/mL, and further 0.005 g/mL or higher.

Regarding the weakly basic anion exchange resins (WA30 and IRA96SB), it was also confirmed that the rate of lactic acid adsorption increased as the concentration of the adsorbent increased, but concurrently the rate of glucose adsorption also tended to increase. The rate of lactic acid adsorption was, however, nearly 1.5 times as large as the rate of glucose adsorption, even at an adsorbent concentration of 0.2 g/mL where the rate of glucose adsorption peaked at 56%. Meanwhile, the rate of lactic acid adsorption increased up to 2.7 times or more as large as the rate of glucose adsorption, at an adsorbent concentration of 0.1 g/mL. The weakly basic anion exchange resin was therefore found to demonstrate further improved adsorption selectivity to lactic acid, at an adsorbent concentration of less than 0.2 g/mL, and further 0.1 g/mL or less.

Meanwhile, it was confirmed regarding the strongly basic anion exchange resins (SA10A, SA20A), that the rate of lactic acid adsorption increased as the concentration of the adsorbent increased, whereas the rate of glucose adsorption almost did not increase. The rate of lactic acid adsorption was nearly 20 times as large as the rate of glucose adsorption, even at an adsorbent concentration of 0.2 g/mL where the rate of glucose adsorption peaked at 4%. From this, the styrene-bound dimethylamine type weakly basic anion exchange resins and the strongly basic anion exchange resins were confirmed to adsorb lactic acid, more selectively than glucose is adsorbed.

Performance Analyses of Adsorbents in Cell Culture Solution

Sodium lactate (FUJIFILM Wako Pure Chemical Corporation) was added to a pluripotent stem cell medium (StemFit AK02N: Ajinomoto Co., Inc.) to prepare a medium having a glucose concentration of 250 mg/mL and a lactic acid concentration of 10 mM. Ten milliliters each of the medium was then dispensed into a plurality of 15 mL tubes (Thermo Fisher Scientific). Each of various adsorbents was then added to the medium in each tube. The adsorbents employed were WA30 and SA10A. The amount of addition (concentration) of the adsorbent was varied among 0.005 g (0.0005 g/mL), 0.05 g (0.00 5g/mL), 0.25 g (0.025 g/mL), 0.5 g (0.05 g/mL), 1.0 g (0.1 g/mL) and 2.0 g (0.2 g/mL). The mixture was then shaken at 37°C and 60 rpm for 24 hours.

After 24 hours, the culture solution and the adsorbent were separated by a 0.22 $\mu$m filter. The lactic acid concentration and the glucose concentration in the cell culture solution were then measured by using a blood gas analyzer (ABL800 FLEX: Radiometer Medial ApS). The rate of lactic acid adsorption and the rate of glucose adsorption of each adsorbent were calculated from the equation above.

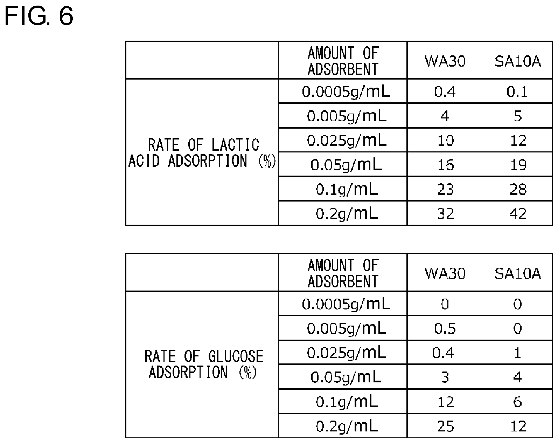

Results are summarized in Fig. 6. Fig. 6 summarizes the rate of lactic acid adsorption and the rate of glucose adsorption of the lactic acid adsorbents in the cell culture solution, when the styrene-bound dimethylamine type weakly basic anion exchange resins and the strongly basic anion exchange resins were used as the lactic acid adsorbents. As summarized in Fig. 6, the styrene-bound dimethylamine type weakly basic anion exchange resin (WA30) and the strongly basic anion exchange resin (SA10A) were confirmed to adsorb lactic acid also in the cell culture solution, although becoming slightly lower than in the water-based solution. Further improved rate of lactic acid adsorption was confirmed to be attainable, particularly at an adsorbent concentration exceeding 0.0005 g/mL, further at 0.005 g/mL or higher, and again further at 0.025 g/mL or more. In addition, each of the styrene-bound dimethylamine type weakly basic anion exchange resin and the strongly basic anion exchange resin demonstrated a rate of glucose adsorption of 25% at the maximum. From this, the styrene-bound dimethylamine-type weakly basic anion exchange resins and the strongly basic anion exchange resins were confirmed to be suitable for removing lactic acid in a medium that contains glucose.

It was confirmed that, also in the cell culture solution, the rate of lactic acid adsorption increased as the adsorbent concentration increased, but concurrently the rate of glucose adsorption also increased. It was however confirmed that the rate of glucose adsorption can be reduced more satisfactorily, if the adsorbent concentration is adjusted to lower than 0.2 g/mL, and preferably to 0.1 g/mL or lower.

[INDUSTRIAL APPLICABILITY]

The present invention is applicable to a lactic acid adsorbent and a method for removing lactic acid.

[REFERENCE SIGNS LIST]

2 container, 4 lactic acid adsorbent, 6 adsorption module, 8 circulation path, 10 culture vessel, 12 pump, 14 culture solution, 16 cell, 18 diaphragm

**Claims**

1. A lactic acid adsorbent comprising at least one substance selected from the group consisting of layered double hydroxide, layered double oxide, weakly basic anion exchange resin having styrene-bound dimethylamino group, and strongly basic anion exchange resin.

2. The lactic acid adsorbent according to claim 1, allowed for contact with a solution containing lactic acid, to adsorb lactic acid in the solution.

3. The lactic acid adsorbent according to claim 2, wherein the solution is a culture solution of cells or microorganisms that contains glucose.

4. The lactic acid adsorbent according to any one of claims 1 to 3, wherein the strongly basic anion exchange resin contains at least one of strongly basic anion exchange resin having a styrene-bound trimethylammonium group or a strongly basic anion exchange resin having a styrene-bound dimethylethanolammonium group.

5. The lactic acid adsorbent according to any one of claims 1 to 4, wherein the weakly basic anion exchange resin is of high porous type or of MR type.

6. The lactic acid adsorbent according to claim 2 or 3, wherein the amount of addition thereof to the solution is more than 0.0005 g/mL and less than 0.2 g/mL.

7. A method for removing lactic acid, the method comprising bringing the lactic acid adsorbent described in any one of claims 1 to 6 in contact with lactic acid.

FIG. 1A

FIG. 1B

FIG. 1C

FIG. 1D

FIG. 2

| ADSORBENT | | SAC | MgO | SiO$_2$ | Cu–Al LDH | Cu–Al LDO | Mg–Al LDO |
|---|---|---|---|---|---|---|---|
| RATE OF LACTIC ACID ADSORPTION (%) | 0.0005g/mL | — | — | — | 4.5 | — | 8.4 |
| | 0.005g/mL | — | — | — | 45.2 | — | 45 |
| | 0.025g/mL | 0 | 0 | 0 | 72.7 | 64.3 | 54.5 |
| | 0.05g/mL | — | — | — | 89.5 | — | 71.3 |
| | 0.1g/mL | — | — | — | 95.4 | — | 80.8 |

| ADSORBENT | | SAC | MgO | SiO$_2$ | Cu–Al LDH | Cu–Al LDO | Mg–Al LDO |
|---|---|---|---|---|---|---|---|
| RATE OF GLUCOSE ADSORPTION (%) | 0.0005g/mL | — | — | — | 1.3 | — | 0 |
| | 0.005g/mL | — | — | — | 0.4 | — | 8.9 |
| | 0.025g/mL | 52.5 | 36.9 | 0 | 5.6 | 4.5 | 9.4 |
| | 0.05g/mL | — | — | — | 18.1 | — | 15.9 |
| | 0.1g/mL | — | — | — | 33.7 | — | 27.3 |

EP 3 848 115 A1

## FIG. 3

| ADSORBENT | | SAC | MgO | SiO₂ | Cu-Al LDH | Cu-Al LDO | Mg-Al LDO |
|---|---|---|---|---|---|---|---|
| RATE OF LACTIC ACID ADSORPTION (%) | 0.0005g/mL | — | — | — | 2 | 1 | 1 |
| | 0.005g/mL | — | — | — | 18 | 13 | 20 |
| | 0.025g/mL | — | — | — | 41 | 24 | 36 |
| | 0.05g/mL | — | — | — | 61 | 46 | 59 |
| | 0.1g/mL | — | — | — | 74 | 63 | 71 |
| | 0.2g/mL | — | — | — | 81 | 70 | 76 |

| ADSORBENT | | SAC | MgO | SiO₂ | Cu-Al LDH | Cu-Al LDO | Mg-Al LDO |
|---|---|---|---|---|---|---|---|
| RATE OF GLUCOSE ADSORPTION (%) | 0.0005g/mL | — | — | — | 1 | 1 | 2 |
| | 0.005g/mL | — | — | — | 5 | 4 | 5 |
| | 0.025g/mL | — | — | — | 10 | 11 | 9 |
| | 0.05g/mL | — | — | — | 15 | 16 | 15 |
| | 0.1g/mL | — | — | — | 17 | 18 | 17 |
| | 0.2g/mL | — | — | — | 23 | 26 | 25 |

EP 3 848 115 A1

FIG. 4A

| ADSORBENT | Mg-Al LDH | Ni-Al LDH |
|---|---|---|
| RATE OF LACTIC ACID ADSORPTION (%) | 67.5 | 72.6 |
| RATE OF GLUCOSE ADSORPTION (%) | 8.7 | 1.1 |

FIG. 4B

| ADSORBENT | Mg-Al LDH | Ni-Al LDH |
|---|---|---|
| RATE OF LACTIC ACID ADSORPTION (%) | 24.0 | 49.8 |
| RATE OF GLUCOSE ADSORPTION (%) | 3.6 | 8.3 |

## FIG. 5A

| ADSORBENT | α-Fe₂O₃ | γ-Fe₂O₃ | Al₂O₃ | Al(OH)₃ | WA20 | WA30 | SA10A | SA20A | IRA67 | IRA96SB |
|---|---|---|---|---|---|---|---|---|---|---|
| RATE OF LACTIC ACID ADSORPTION (%) | 0 | 11 | 0 | 0 | 5 | 49 | 70 | 75 | 2.4 | 23 |

## FIG. 5B

| AMOUNT OF ADSORBENT | WA30 | IRA96SB | SA10A | SA20A |
|---|---|---|---|---|
| 0.0005g/mL | 3 | 7 | 8 | 7 |
| 0.005g/mL | 11 | 11 | 35 | 32 |
| 0.025g/mL | 49 | 23 | 70 | 75 |
| 0.1g/mL | 60 | 38 | 67 | 76 |
| 0.2g/mL | 80 | 54 | 79 | 84 |

RATE OF LACTIC ACID ADSORPTION (%)

| AMOUNT OF ADSORBENT | WA30 | IRA96SB | SA10A | SA20A |
|---|---|---|---|---|
| 0.0005g/mL | 0.2 | 4 | 1 | 4 |
| 0.005g/mL | 0.3 | 1 | 2 | 2 |
| 0.025g/mL | 17 | 7 | 3 | 4 |
| 0.1g/mL | 18 | 14 | 4 | 4 |
| 0.2g/mL | 56 | 35 | 4 | 3 |

RATE OF GLUCOSE ADSORPTION (%)

## FIG. 6

| | AMOUNT OF ADSORBENT | WA30 | SA10A |
|---|---|---|---|
| RATE OF LACTIC ACID ADSORPTION (%) | 0.0005g/mL | 0.4 | 0.1 |
| | 0.005g/mL | 4 | 5 |
| | 0.025g/mL | 10 | 12 |
| | 0.05g/mL | 16 | 19 |
| | 0.1g/mL | 23 | 28 |
| | 0.2g/mL | 32 | 42 |

| | AMOUNT OF ADSORBENT | WA30 | SA10A |
|---|---|---|---|
| RATE OF GLUCOSE ADSORPTION (%) | 0.0005g/mL | 0 | 0 |
| | 0.005g/mL | 0.5 | 0 |
| | 0.025g/mL | 0.4 | 1 |
| | 0.05g/mL | 3 | 4 |
| | 0.1g/mL | 12 | 6 |
| | 0.2g/mL | 25 | 12 |

**INTERNATIONAL SEARCH REPORT**

International application No.

PCT/JP2019/033125

A. CLASSIFICATION OF SUBJECT MATTER
Int.Cl. B01J20/06(2006.01)i, B01D15/00(2006.01)i

According to International Patent Classification (IPC) or to both national classification and IPC

B. FIELDS SEARCHED

Minimum documentation searched (classification system followed by classification symbols)
Int.Cl. B01J20/06, B01D15/00

Documentation searched other than minimum documentation to the extent that such documents are included in the fields searched
Published examined utility model applications of Japan 1922–1996
Published unexamined utility model applications of Japan 1971–2019
Registered utility model specifications of Japan 1996–2019
Published registered utility model applications of Japan 1994–2019

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2014-39505 A (ASAHI GLASS CO., LTD.) 06 March 2014, claims, paragraphs [0001]-[0002], [0018], [0029], [0032], [0040]-[0051] (Family: none) | 1-4, 6-7<br>6-7 |
| X<br>Y | JP 63-188632 A (SHIMADZU CORPORATION) 04 August 1988, claims, page 2, lower left column, line 17 to page 4, upper left column, line 1 (Family: none) | 1-4, 6-7<br>6 |
| X<br>Y | JP 2006-212617 A (YOSHIDA, Hiroyuki) 17 August 2006, paragraphs [0034], [0038]-[0044] (Family: none) | 1-2, 4, 6-7<br>6 |

☒ Further documents are listed in the continuation of Box C.   ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
07 November 2019 (07.11.2019)

Date of mailing of the international search report
19 November 2019 (19.11.2019)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

**INTERNATIONAL SEARCH REPORT**

International application No.

PCT/JP2019/033125

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2001-95487 A (ORGANO CORP.) 10 April 2001,<br>claims, paragraphs [0007]-[0026] (Family: none) | 1-2, 5-7<br>6 |
| X<br>Y | JP 11-32724 A (SNOW BRAND MILK PRODUCTS CO., LTD.)<br>09 February 1999, claims, paragraphs [0006],<br>[0009]-[0027] (Family: none) | 1-2, 5-7<br>6 |
| X<br>Y | JP 58-214338 A (KYOWA CHEMICAL INDUSTRY CO., LTD.)<br>13 December 1983, claims, page 2, lower left<br>column, line 15 to page 4, lower left column, line<br>9 & US 4458030 A, claims, column 2, line 13 to<br>column 4, line 60 | 1-2, 6-7<br>6-7 |
| X<br>Y | JP 2017-567 A (TOAGOSEI CO., LTD.) 5 January 2017,<br>paragraphs [0018], [0032] (Family: none) | 1-2, 6-7<br>6-7 |

Form PCT/ISA/210 (continuation of second sheet) (January 2015)

**REFERENCES CITED IN THE DESCRIPTION**

**Patent documents cited in the description**

- WO 2015122528 A